# DEMANDE DE BREVET EUROPEEN

(11) **EP 3 845 556 A1**
(43) Date de publication de la demande: **07.07.2021**
(21) Numéro de dépôt: 19306799.8
(22) Date de dépôt: 31.12.2019
(51) Int. Cl.: C07K 14/54, A61K 38/20, A61K 39/00

(54) **CONJUGUE IMMUNOGENE DESTINE A INDUIRE UNE REPONSE IMMUNITAIRE DIRIGEE CONTRE L INTERLEUKINE-6**

(71) Demandeur: Peptinov SAS, 75009 Paris (FR); Conservatoire National des Arts et Métiers, 75003 Paris (FR)
(72) Inventeur: DESALLAIS, Lucille, 95170 Deuil-La-Barre (FR); SALLES, Jean-Pierre, 13510 Eguilles (FR); ZAGURY, Jean-François, 78110 Le Vesinet (FR)
(74) Mandataire: Vial, Lionel

(57) **Abrégé**

La présente invention concerne un conjugué immunogène comprenant :
- une protéine porteuse et
- au moins un polypeptide ayant au plus 100 acides aminés comprenant une séquence de 5 à 50 acides aminés de l'interleukine 6 (IL-6) ou du récepteur de l'IL-6 (IL-6R), ou une séquence variante présentant au moins 75% d'identité avec la séquence de 5 à 50 acides aminés de l'IL-6 ou de l'IL-6R,
dans lequel le polypeptide est lié de manière covalente à la protéine porteuse et la protéine porteuse est une toxine diphtérique mutante non-toxique.

SEQUENCE LISTING

<110>  PEPTINOV SAS
       CONSERVATOIRE NATIONAL DES ARTS ET METIERS

<120>  CONJUGUE IMMUNOGENE DESTINE A INDUIRE UNE REPONSE IMMUNITAIRE
       DIRIGEE CONTRE L'INTERLEUKINE-6

<130>  B000112EP

<160>  28

<170>  PatentIn version 3.5

<210>  1
<211>  212
<212>  PRT
<213>  Homo sapiens

<400>  1

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
        180                 185                 190

Leu Ile Leu Arg Ser Phe Lys Glu Phe Leu Gln Ser Ser Leu Arg Ala
        195                 200                 205

Leu Arg Gln Met
    210

<210>  2
<211>  468
<212>  PRT
<213>  Homo sapiens

<400>  2

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg
            20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala Glu Asn Glu Val Ser Thr Pro Met Gln Ala Leu Thr Thr
                325                 330                 335

Asn Lys Asp Asp Asp Asn Ile Leu Phe Arg Asp Ser Ala Asn Ala Thr
            340                 345                 350

Ser Leu Pro Val Gln Asp Ser Ser Ser Val Pro Leu Pro Thr Phe Leu
        355                 360                 365

Val Ala Gly Gly Ser Leu Ala Phe Gly Thr Leu Leu Cys Ile Ala Ile
    370                 375                 380

Val Leu Arg Phe Lys Lys Thr Trp Lys Leu Arg Ala Leu Lys Glu Gly
385                 390                 395                 400

Lys Thr Ser Met His Pro Pro Tyr Ser Leu Gly Gln Leu Val Pro Glu
                405                 410                 415

Arg Pro Arg Pro Thr Pro Val Leu Val Pro Leu Ile Ser Pro Pro Val

420                 425                 430

Ser Pro Ser Ser Leu Gly Ser Asp Asn Thr Ser Ser His Asn Arg Pro
        435                 440                 445

Asp Ala Arg Asp Pro Arg Ser Pro Tyr Asp Ile Ser Asn Thr Asp Tyr
    450                 455                 460

Phe Phe Pro Arg
465

<210>  3
<211>  21
<212>  PRT
<213>  Artificial sequence

<220>
<223>  Séquence d'IL-6

<400>  3

Arg Tyr Ile Ile Asp Gly Ile Ser Ala Leu Arg Lys Glu Thr Cys Asn
1               5                   10                  15

Lys Ser Asn Met Cys
            20

<210>  4
<211>  22
<212>  PRT
<213>  Artificial sequence

<220>
<223>  Séquence d'IL-6

<400>  4

Asn Lys Ser Asn Met Cys Glu Ser Ser Lys Glu Ala Leu Ala Glu Asn
1               5                   10                  15

Asn Leu Asn Leu Pro Lys
            20

<210>  5
<211>  16
<212>  PRT
<213>  Artificial sequence

<220>
<223>  Séquence d'IL-6

<400>  5

Ala Glu Lys Asp Gly Cys Phe Gln Ser Gly Phe Asn Glu Glu Thr Cys
1               5                   10                  15

<210>  6
<211>  20
<212>  PRT
<213>  Artificial sequence

<220>
<223>  Séquence d'IL-6

<400>  6

Phe Glu Val Tyr Leu Glu Tyr Leu Gln Asn Arg Phe Glu Ser Ser Glu
1               5                   10                  15

Glu Gln Ala Arg
            20

<210>  7
<211>  18
<212>  PRT
<213>  Artificial sequence

<220>
<223>  Sequence d'IL-6

<400>  7

Asn Ala Ser Leu Leu Thr Lys Leu Gln Ala Gln Asn Gln Trp Leu Gln
1               5                   10                  15

Asp Met

<210>  8
<211>  17
<212>  PRT
<213>  Artificial sequence

<220>
<223>  Séquence d'IL-6

<400>  8

Arg Ser Phe Lys Glu Phe Leu Gln Ser Ser Leu Arg Ala Leu Arg Gln
1               5                   10                  15

Met

<210>  9
<211>  21
<212>  PRT
<213>  Artificial sequence

<220>
<223>  Séquence d'IL-6

<400>  9

Val Leu Trp Glu Ile Val Glu Met Arg Lys Glu Leu Cys Asn Gly Asn
1               5                   10                  15

Ser Asp Cys Met Asn
            20

<210>  10
<211>  22
<212>  PRT
<213>  Artificial sequence

<220>
<223>  Séquence d'IL-6

<400>  10

Asn Ser Asp Cys Met Asn Asn Asp Asp Ala Leu Ala Glu Asn Asn Leu
1               5                   10                  15

Lys Leu Pro Glu Ile Gly
            20

<210>  11
<211>  16
<212>  PRT
<213>  Artificial sequence

<220>
<223>  Séquence d'IL-6

<400>  11

Asn Asp Gly Cys Tyr Gln Thr Gly Tyr Asn Gln Glu Ile Cys Leu Leu
1               5                   10                  15

<210>  12
<211>  20
<212>  PRT
<213>  Artificial sequence

<220>
<223>  Séquence d'IL-6

<400>  12

Ser Tyr Leu Glu Tyr Met Lys Asn Asn Leu Lys Asp Asn Lys Lys Asp
1               5                   10                  15

Lys Ala Arg Val
            20

<210>  13
<211>  18

<212>  PRT
<213>  Artificial sequence

<220>
<223>  Séquence d'IL-6

<400>  13

Ala Leu Leu Thr Asp Lys Leu Glu Ser Gln Lys Glu Trp Leu Arg Thr
1               5                   10                  15

Lys Thr

<210>  14
<211>  16
<212>  PRT
<213>  Artificial sequence

<220>
<223>  Séquence d'IL-6

<400>  14

Ser Leu Glu Glu Phe Leu Lys Val Thr Leu Arg Ser Thr Arg Gln Thr
1               5                   10                  15

<210>  15
<211>  4
<212>  PRT
<213>  Artificial sequence

<220>
<223>  Lien peptidique

<220>
<221>  MISC_FEATURE
<222>  (4)..(4)
<223>  Cys (C), Tyr (Y), Lys (K)

<400>  15

Glu Gly Glu Xaa
1

<210>  16
<211>  535
<212>  PRT
<213>  Corynebacterium diphtheriae

<400>  16

Gly Ala Asp Asp Val Val Asp Ser Ser Lys Ser Phe Val Met Glu Asn
1               5                   10                  15

Phe Ser Ser Tyr His Gly Thr Lys Pro Gly Tyr Val Asp Ser Ile Gln
            20                  25                  30

Lys Gly Ile Gln Lys Pro Lys Ser Gly Thr Gln Gly Asn Tyr Asp Asp
        35                  40                  45

Asp Trp Lys Glu Phe Tyr Ser Thr Asp Asn Lys Tyr Asp Ala Ala Gly
    50                  55                  60

Tyr Ser Val Asp Asn Glu Asn Pro Leu Ser Gly Lys Ala Gly Gly Val
65                  70                  75                  80

Val Lys Val Thr Tyr Pro Gly Leu Thr Lys Val Leu Ala Leu Lys Val
                85                  90                  95

Asp Asn Ala Glu Thr Ile Lys Lys Glu Leu Gly Leu Ser Leu Thr Glu
            100                 105                 110

Pro Leu Met Glu Gln Val Gly Thr Glu Glu Phe Ile Lys Arg Phe Gly
        115                 120                 125

Asp Gly Ala Ser Arg Val Val Leu Ser Leu Pro Phe Ala Glu Gly Ser
    130                 135                 140

Ser Ser Val Glu Tyr Ile Asn Asn Trp Glu Gln Ala Lys Ala Leu Ser
145                 150                 155                 160

Val Glu Leu Glu Ile Asn Phe Glu Thr Arg Gly Lys Arg Gly Gln Asp
                165                 170                 175

Ala Met Tyr Glu Tyr Met Ala Gln Ala Cys Ala Gly Asn Arg Val Arg
            180                 185                 190

Arg Ser Val Gly Ser Ser Leu Ser Cys Ile Asn Leu Asp Trp Asp Val
        195                 200                 205

Ile Arg Asp Lys Thr Lys Thr Lys Ile Glu Ser Leu Lys Glu His Gly
    210                 215                 220

Pro Ile Lys Asn Lys Met Ser Glu Ser Pro Asn Lys Thr Val Ser Glu
225                 230                 235                 240

Glu Lys Ala Lys Gln Tyr Leu Glu Glu Phe His Gln Thr Ala Leu Glu
                245                 250                 255

His Pro Glu Leu Ser Glu Leu Lys Thr Val Thr Gly Thr Asn Pro Val
            260                 265                 270

Phe Ala Gly Ala Asn Tyr Ala Ala Trp Ala Val Asn Val Ala Gln Val

        275                 280                 285

Ile Asp Ser Glu Thr Ala Asp Asn Leu Glu Lys Thr Thr Ala Ala Leu
    290                 295                 300

Ser Ile Leu Pro Gly Ile Gly Ser Val Met Gly Ile Ala Asp Gly Ala
305                 310                 315                 320

Val His His Asn Thr Glu Glu Ile Val Ala Gln Ser Ile Ala Leu Ser
                325                 330                 335

Ser Leu Met Val Ala Gln Ala Ile Pro Leu Val Gly Glu Leu Val Asp
            340                 345                 350

Ile Gly Phe Ala Ala Tyr Asn Phe Val Glu Ser Ile Ile Asn Leu Phe
        355                 360                 365

Gln Val Val His Asn Ser Tyr Asn Arg Pro Ala Tyr Ser Pro Gly His
    370                 375                 380

Lys Thr Gln Pro Phe Leu His Asp Gly Tyr Ala Val Ser Trp Asn Thr
385                 390                 395                 400

Val Glu Asp Ser Ile Ile Arg Thr Gly Phe Gln Gly Glu Ser Gly His
                405                 410                 415

Asp Ile Lys Ile Thr Ala Glu Asn Thr Pro Leu Pro Ile Ala Gly Val
            420                 425                 430

Leu Leu Pro Thr Ile Pro Gly Lys Leu Asp Val Asn Lys Ser Lys Thr
        435                 440                 445

His Ile Ser Val Asn Gly Arg Lys Ile Arg Met Arg Cys Arg Ala Ile
    450                 455                 460

Asp Gly Asp Val Thr Phe Cys Arg Pro Lys Ser Pro Val Tyr Val Gly
465                 470                 475                 480

Asn Gly Val His Ala Asn Leu His Val Ala Phe His Arg Ser Ser Ser
                485                 490                 495

Glu Lys Ile His Ser Asn Glu Ile Ser Ser Asp Ser Ile Gly Val Leu
            500                 505                 510

Gly Tyr Gln Lys Thr Val Asp His Thr Lys Val Asn Ser Lys Leu Ser
        515                 520                 525

Leu Phe Phe Glu Ile Lys Ser
    530                 535


<210>  17
<211>  15
<212>  PRT
<213>  Artificial sequence

<220>
<223>  Variant de séquence d'IL-6

<400>  17

Glu Ser Lys Glu Ala Leu Ala Glu Asn Asn Leu Asn Leu Pro Lys
1               5                   10                  15


<210>  18
<211>  16
<212>  PRT
<213>  Artificial sequence

<220>
<223>  Variant de séquence d'IL-6

<400>  18

Glu Glu Ser Lys Glu Ala Leu Ala Glu Asn Asn Leu Asn Leu Pro Lys
1               5                   10                  15


<210>  19
<211>  17
<212>  PRT
<213>  Artificial sequence

<220>
<223>  Variant de séquence d'IL-6

<400>  19

Glu Glu Ser Lys Glu Ala Leu Ala Glu Asn Asn Leu Asn Leu Pro Lys
1               5                   10                  15


Cys



<210>  20
<211>  18
<212>  PRT
<213>  Artificial sequence

<220>
<223>  Variant de séquence d'IL-6

<400>  20

Ala Glu Glu Ser Lys Glu Ala Leu Ala Glu Asn Asn Leu Asn Leu Pro
1               5                   10                  15

Lys Cys

<210> 21
<211> 18
<212> PRT
<213> Artificial sequence

<220>
<223> Variant de séquence d'IL-6

<400> 21

Cys Glu Glu Ser Lys Glu Ala Leu Ala Glu Asn Asn Leu Asn Leu Pro
1               5                   10                  15

Lys Cys

<210> 22
<211> 19
<212> PRT
<213> Artificial sequence

<220>
<223> Polypeptide selon l'invention

<400> 22

Cys Glu Ser Ser Lys Glu Ala Leu Ala Glu Asn Asn Leu Asn Leu Pro
1               5                   10                  15

Lys Cys Tyr

<210> 23
<211> 15
<212> PRT
<213> Artificial sequence

<220>
<223> Polypeptide selon l'invention

<400> 23

Leu Thr Lys Leu Gln Ala Gln Asn Gln Trp Leu Gln Asp Met Cys
1               5                   10                  15

<210> 24
<211> 25
<212> PRT
<213> Artificial sequence

<220>
<223> Polypeptide selon l'invention

<400>  24

Cys Ile Asp Lys Gln Ile Arg Tyr Ile Ile Asp Gly Ile Ser Ala Leu
1               5                   10                  15

Arg Lys Glu Thr Cys Glu Gly Glu Cys
            20                  25

<210>  25
<211>  14
<212>  PRT
<213>  Artificial sequence

<220>
<223>  Polypeptide selon l'invention

<400>  25

Cys Leu Gln Ala Gln Asn Gln Trp Leu Gln Asp Met Cys Tyr
1               5                   10

<210>  26
<211>  17
<212>  PRT
<213>  Artificial sequence

<220>
<223>  Polypeptide selon l'invention

<400>  26

Cys Leu Gln Ala Gln Asn Gln Trp Leu Gln Asp Met Cys Glu Gly Glu
1               5                   10                  15

Tyr

<210>  27
<211>  11
<212>  PRT
<213>  Artificial sequence

<220>
<223>  Polypeptide selon l'invention

<400>  27

Cys Phe Gln Ser Gly Phe Asn Glu Glu Thr Cys
1               5                   10

<210>  28
<211>  15
<212>  PRT
<213>  Artificial sequence

<220>
<223>  Polypeptide selon l'invention

<400>  28

Cys Phe Gln Ser Gly Phe Asn Glu Glu Thr Cys Glu Gly Glu Tyr
1               5                   10                  15

## Description

### Domaine de l'invention

La présente invention a pour objet un conjugué immunogène, une composition pharmaceutique, notamment vaccinale, le comprenant, et son utilisation dans une méthode de prévention ou de traitement des maladies liées à une surexpression ou surproduction de l'interleukine-6.

### Arrière-plan technique

L'interleukine-6 (IL-6) est une cytokine essentielle impliquée notamment la régulation de la prolifération et de la différenciation des cellules immunitaires.

L'IL-6 est fortement surproduite lors des processus inflammatoires, et cette surproduction est observée dans de nombreuses maladies comme par exemple les infections, les maladies inflammatoires aigües ou chroniques, et aussi le cancer.

L'IL-6 apparaît ainsi comme le signal moteur dans plusieurs maladies inflammatoires. Des études précliniques dans des modèles animaux de maladies humaines ont montré que le blocage de l'activité de l'IL-6 atténue les symptômes ou même empêche complètement l'apparition de la maladie.

La cascade de signalisation de l'IL-6 offre plusieurs alternatives pour une intervention thérapeutique, allant des produits biologiques qui bloquent la cytokine ou son récepteur à l'extérieur de la cellule aux petites molécules chimiques qui ciblent les kinases et les facteurs de transcription impliqués à l'intérieur de la cellule. Il est intéressant de noter que chez les patients, les anticorps qui ciblent l'IL-6 peuvent être utilisés en quantités nettement inférieures par rapport aux anticorps qui bloquent le récepteur de l'IL-6 (IL-6R). En effet, le récepteur soluble à l'IL-6 (sIL-6R) est présent dans le sérum à des concentrations élevées et comme ces récepteurs solubles captent aussi les anticorps neutralisants anti-IL-6R, il faut beaucoup de ces derniers pour qu'ils puissent aussi bloquer les récepteurs membranaires IL-6R à la surface des cellules et ainsi empêcher la signalisation de l'IL-6.

Les concentrations d'IL-6 sont relativement faibles chez les individus en bonne santé (quelques picogrammes par millilitre de sérum) et les anticorps ciblant directement la cytokine IL-6 n'ont besoin de capturer que les molécules d'IL-6 nouvellement synthétisées et libérées pour être actifs. Le blocage direct de l'IL-6 n'interfère pas avec les autres cytokines pouvant transmettre des signaux via l'IL-6R et offre donc une inhibition bien spécifique de l'IL-6.

Depuis quelques années, plusieurs classes de produits thérapeutiques ciblant les composants de la voie de signalisation de l'IL-6 ont été développées. L'efficacité des interventions thérapeutiques neutralisant l'IL-6 ou interférant avec sa signalisation a démontré le rôle délétère majeur de l'IL-6 dans un certain nombre de maladies.

Ainsi, le tocilizumab bloqueur de IL-6R, premier anticorps monoclonal développé contre la voie IL-6, est aujourd'hui approuvé pour le traitement de la Polyarthrite rhumatoïde (PR) active de l'adulte modérée à sévère. D'autres anticorps monoclonaux ciblant IL-6R sont en développement : le sarilumab, approuvé dans la polyarthrite rhumatoïde; le NI-1201, actuellement en phase d'essais précliniques; le vobarilizumab, actuellement en essais cliniques de phase II pour le traitement de la polyarthrite rhumatoïde et du lupus érythémateux systémique.

Plusieurs anticorps monoclonaux ciblant directement l'IL-6 ont aussi été développés, on peut ainsi citer : le sirukumab, destiné au traitement de la polyarthrite rhumatoïde, de la dépression et du lupus néphrétique (phase II) ; l'olokizumab, destiné au traitement de la polyarthrite rhumatoïde et de la maladie de Crohn ; le clazakizumab, destiné au traitement du rejet de transplantation d'organes; le siltuximab, indiqué dans le traitement de la maladie de Castleman et destiné par ailleurs au traitement du myélome multiple.

Toutefois, tous ces traitements d'immunothérapie passive, c'est-à-dire impliquant l'administration d'anticorps, notamment monoclonaux, nécessitent des administrations assez fréquentes et au long cours, ce qui entraîne des contraintes importantes tant en ce qui concerne le confort du patient que les coûts.

Afin de surmonter ces inconvénients, une approche de prévention et de traitement des maladies liées à l'IL-6 basée sur l'immunothérapie active (ou vaccinale) a été développée. Cette approche repose sur l'induction d'une réponse immunitaire humorale à l'aide de polypeptides immunogènes dérivés de l'IL-6 aboutissant à la production d'anticorps anti-IL-6 par le patient lui-même.

Desallais et al. (2016) Sci. Rep. 6:19549 décrivent ainsi la production d'anticorps anti-IL-6 humaine neutralisants chez des singes cynomolgus auxquels a été administré un polypeptide dérivé de l'IL-6. Toutefois, les taux d'anticorps obtenus restaient modérés.

Un objectif de la présente invention est donc d'améliorer la production d'anticorps anti-IL-6 en immunothérapie active.

### Résumé de l'invention

La présente invention découle de la mise en évidence inattendue, par les inventeurs, que la conjugaison de polypeptides dérivés de l'IL-6 à la protéine porteuse CRM-197 permettait d'augmenter la production d'anticorps anti-IL-6 chez l'individu auquel le conjugué est administré par rapport à d'autres protéines porteuses.

Ainsi, la présente invention concerne un conjugué immunogène comprenant :
- une protéine porteuse et
- au moins un polypeptide ayant au plus 100 acides aminés comprenant une séquence de 5 à 50 acides aminés de l'interleukine 6 (IL-6) ou du récepteur de l'interleukine 6 (IL-6R), ou une séquence variante présentant au moins 75% d'identité avec la séquence de 5 à 50 acides aminés de l'IL-6 ou l'IL-6R,
dans lequel l'au moins un polypeptide est lié de manière covalente à la protéine porteuse et la protéine porteuse est une toxine diphtérique mutante non-toxique.

Dans un mode de réalisation particulier du conjugué immunogène tel que défini ci-dessus, le polypeptide est lié à la protéine porteuse via un agent de couplage non peptidique.

La présente invention concerne également le conjugué immunogène tel que défini ci-dessus, pour une utilisation dans une méthode de traitement thérapeutique, pour une utilisation dans une méthode de prévention ou de traitement d'une maladie liée à une surproduction ou une surexpression de l'IL-6, ou pour une utilisation dans une méthode de vaccination contre l'IL-6 ou l'IL-6R, ou dans une méthode d'induction d'une réponse immunitaire contre l'IL-6 ou l'IL-6R, chez un individu.

La présente invention concerne également une méthode de prévention ou de traitement d'une maladie liée à une surexpression ou une surproduction de l'IL-6 chez un individu, dans laquelle on administre à l'individu une quantité prophylactiquement ou thérapeutiquement efficace d'un conjugué immunogène tel que défini ci-dessus.

La présente invention concerne également une méthode de vaccination contre l'IL-6 ou l'IL-6R ou d'induction d'une réponse immunitaire contre l'IL-6 ou l'IL-6R chez un individu, dans laquelle on administre à l'individu une quantité efficace d'un conjugué immunogène tel que défini ci-dessus.

La présente invention concerne également une composition pharmaceutique, notamment vaccinale, comprenant à titre de substance active au moins un conjugué immunogène tel que défini ci-dessus, éventuellement en association avec au moins un véhicule et/ou excipient pharmaceutiquement acceptable.

Dans un mode de réalisation particulier de l'invention, la composition pharmaceutique, notamment vaccinale, tel que définie ci-dessus comprend en outre au moins un adjuvant.

La présente invention concerne également la composition pharmaceutique, notamment vaccinale, tel que définie ci-dessus, pour une utilisation dans une méthode de traitement thérapeutique, pour une utilisation dans une méthode de prévention ou de traitement d'une maladie liée à une surexpression ou une surproduction de l'IL-6, ou pour une utilisation dans une méthode de vaccination contre l'IL-6 ou l'IL-6R, ou dans une méthode d'induction d'une réponse immunitaire contre l'IL-6 ou l'IL-6R, chez un individu.

La présente invention concerne également une méthode de prévention ou de traitement d'une maladie liée à une surexpression de l'IL-6 chez un individu, dans laquelle on administre à l'individu une quantité prophylactiquement ou thérapeutiquement efficace d'une composition pharmaceutique telle que définie ci-dessus.

La présente invention concerne également une méthode de vaccination contre l'IL-6 ou l'IL-6R ou d'induction d'une réponse immunitaire contre l'IL-6 ou l'IL-6R chez un individu, dans laquelle on administre à l'individu une quantité efficace d'une composition pharmaceutique telle que définie ci-dessus.

La présente invention concerne également un procédé de préparation d'un conjugué immunogène tel que défini ci-dessus, comprenant une étape de liaison covalente d'au moins un polypeptide ayant au plus 100 acides aminés comprenant une séquence de 5 à 50 acides aminés de l'interleukine 6 (IL-6) ou du récepteur de l'IL-6 (IL-6R), ou une séquence variante présentant au moins 75% d'identité avec la séquence de 5 à 50 acides aminés de l'IL-6 ou de l'IL-6R, avec une protéine porteuse qui est une toxine diphtérique mutante non-toxique.

### Description détaillée

A titre préliminaire, on rappellera que l'expression « consistant en » signifie « constitué de », c'est-à-dire que lorsqu'un objet « consiste en » un élément ou plusieurs éléments, l'objet ne peut pas comprendre d'autres éléments que ceux mentionnés. *A contrario,* le terme « comprenant » signifie « incluant », « contenant » ou « englobant », c'est-à-dire que lorsqu'un objet « comprend » un élément ou plusieurs éléments, d'autres éléments que ceux mentionnés peuvent également être compris dans l'objet. Autrement dit, lorsqu'un objet « comprend » un élément ou plusieurs éléments, il est constitué du ou des plusieurs éléments et éventuellement d'autres éléments que ceux-ci.

### Polypeptide

### Définitions

L'interleukine 6 (IL-6), également parfois nommée facteur de stimulation des lymphocytes B 2 *(B-cell stimulatory factor* 2, *BSF-2*)*,* facteur de différenciation des lymphocytes T cytotoxiques (*CTL differentiation factor, CDF),* facteur de croissance des hybridomes, ou interferon β-2 (IFN-β-2) est bien connue de l'homme du métier. De nombreuses séquences d'IL-6 provenant de diverses espèces animales sont disponibles dans les bases de données de séquence. A titre d'exemple, une IL-6 humaine est décrite dans la base de données UniProt/Swissprot sous la référence P05231 (SEQ ID NO : 1). A titre d'exemple également la sous-unité alpha du récepteur de l'IL-6 humaine est décrit dans la base de donnée UniProt/Swissprot sous la référence sous la référence P08887 (SEQ ID NO : 2).

Tels qu'utilisés ici, les termes « peptide » et « polypeptide » sont utilisés dans leur sens le plus large pour désigner une molécule de deux résidus d'acides aminés, ou plus. Les résidus d'acides aminés peuvent être liés par des liaisons peptidiques, ou en variante par d'autres liaisons, par exemple ester, éther, etc., Toutefois, les résidus d'acides aminés sont de préférence liés entre par des liaisons peptidiques.

Telles qu'utilisées ici, les expressions « acide aminé» et « résidu d'acide aminé» englobent les acides aminés naturels et non naturels ou synthétiques, y compris les formes D et L, et les analogues d'acides aminés. Un « analogue d'acide aminé » doit être compris comme un acide aminé d'origine non naturelle qui diffère d'un acide aminé d'origine naturel correspondant sur un ou plusieurs atomes. Par exemple, un analogue d'acide aminé de la cystéine peut être l'homocystéine. Toutefois, les acides aminés ou les résidus d'acides aminés sont de préférence naturels.

Le polypeptide selon l'invention est tel qu'il doit permettre d'éliciter une réponse immunitaire dirigée contre l'IL-6 ou l'IL-6R ; c'est-à-dire que l'administration d'un tel polypeptide lié à une protéine porteuse, comme la protéine CRM-197 ou la KLH par exemple, à un animal, tel qu'une souris, un rat ou un lapin par exemple, provoque la production d'anticorps dirigés contre l'IL-6 ou l'IL-6R. Le polypeptide selon l'invention est donc immunogène et comprend un ou plusieurs épitopes de l'IL-6 ou de l'IL-6R. L'homme du métier sait bien comment déterminer si un anticorps est dirigé contre l'IL-6 ou l'IL-6R, notamment en mettant en œuvre un test ELISA.

Comme on l'entend ici, le pourcentage d'identité entre deux séquences peptidiques peut être déterminé en réalisant un alignement optimal sur toute la longueur des séquences, en déterminant le nombre de positions alignées pour lesquelles les acides aminés sont identiques dans chaque séquence et en divisant ce nombre par le nombre total d'acides aminés dans la plus longue des deux séquences. L'alignement optimal est celui qui donne le pourcentage d'identité le plus élevé entre les deux séquences.

Comme on l'entend ici les termes « surproduction » et « surexpression » de l'IL-6 sont considérés équivalents et signifient que l'IL-6 est présente dans l'organisme d'un individu à traiter dans une concentration au-delà de la normale ou en une concentration non physiologique ou pathologique.

### Longueur

Le polypeptide selon l'invention comprend de préférence au plus 100, 90, 80, 70, 60, 50, 40, 30, 25, 24, 23, 22, 21 ou 20 acides aminés. De préférence, le selon l'invention comprend au moins 5, 6, 7, 8, 9, 10, 12, 15, 18, 20, 40, 50, 60, 70, 80 ou 90 acides aminés. De préférence, le polypeptide selon l'invention comprend de 10 à 40 acides aminés, plus préférablement de 15 à 35 acides aminés.

### IL-6

De préférence, l'IL-6 selon l'invention est sélectionné dans le groupe constitué de l'IL-6 humaine (hIL-6), de l'IL-6 de souris, de l'IL-6 de singe, en particulier de macaque, de l'IL-6 de cheval, de l'IL-6 de chien, de l'IL-6 de chat, ou de l'IL6 de lapin. De manière particulièrement préférée l'IL-6 selon l'invention est l'IL-6 humaine.

### Séquence

De préférence, le polypeptide selon l'invention comprend, ou est constitué de, une séquence d'au moins 5, 6, 7, 8, 9, 10, 11 ou 12 acides aminés de l'IL-6 ou de l'IL-6R. De préférence, le polypeptide selon l'invention comprend, ou est constitué de, une séquence d'au plus 50, 45, 40, 35, 30, 25, 20, 19, 18, 17, 16, 15, 14, 13, 12, 11 ou 10 acides aminés de l'IL-6 ou de l'IL-6R. De préférence, le polypeptide selon l'invention comprend, ou est constitué de, une séquence de 6 à 40, de 7 à 35, ou de 8 à 30 acides aminés de l'IL-6 ou de l'IL-6R.

De préférence, le polypeptide selon l'invention comprend, ou est constitué de, une séquence de 7 à 35 acides aminés de l'IL-6 ou de l'IL-6R, ou une séquence variante présentant au moins 90% d'identité avec la séquence de 7 à 35 acides aminés de l'IL-6 ou de l'IL-6R.

Le polypeptide selon l'invention peut notamment être tel que décrit dans la demande internationale WO2013/021284, qui est incorporée ici par référence.

De préférence, la séquence d'acides aminés de l'IL-6 ou de l'IL-6R selon l'invention comprend au moins 5, 6, 7, 8, 9, 10, 11, ou 12 acides aminés, ou la totalité, des séquences 58-78, 73-94, 96-111, 122-141, ou 172-189 de l'IL-6. De préférence, la séquence d'acides aminés de l'IL-6 ou de l'IL-6R selon l'invention est constituée de 5, 6, 7, 8, 9, 10, 11, ou 12 acides aminés, ou de la totalité, des séquences 58-78, 73-94, 96-111, 122-141, ou 172-189 de l'IL-6.

De préférence, la séquence d'acides aminés de l'IL-6 ou de l'IL-6 selon l'invention comprend au plus 21, 20, 19, 18, 17, 16, 15, 14 ou 13 acides aminés des séquences 58-78, 73-94, 96-111, 122-141, ou 172-189 de l'IL-6.

La numérotation ci-dessus est basée sur la séquence de référence contenant le peptide signal, et présentée à titre d'exemple pour l'IL-6 humaine et l'IL-6 murine :
- IL-6 humaine (hIL-6) :
   Séquence 58 à 78: RYIIDGISALRKETCNKSNMC (SEQ ID NO : 3)
   Séquence 73 à 94 : NKSNMCESSKEALAENNLNLPK (SEQ ID NO : 4)
   Séquence 96 à 111 : AEKDGCFQSGFNEETC (SEQ ID NO : 5)
   Séquence 122 à 141 : FEVYLEYLQNRFESSEEQAR (SEQ ID NO : 6)
   Séquence 172 à 189 : NASLLTKLQAQNQWLQDM (SEQ ID NO : 7)
   Séquence 196 à 212 : RSFKEFLQSSLRALRQM (SEQ ID NO : 8)
- IL-6 murine (mIL-6) :
   Séquence 58 à 78: VLWEIVEMRKELCNGNSDCMN (SEQ ID NO : 9)
   Séquence 73 à 94 : NSDCMNNDDALAENNLKLPEIG (SEQ ID NO : 10)
   Séquence 96 à 111 : NDGCYQTGYNQEICLL (SEQ ID NO : 11)
   Séquence 122 à 141 : SYLEYMKNNLKDNKKDKARV (SEQ ID NO : 12)
   Séquence 172 à 189 : ALLTDKLESQKEWLRTKT (SEQ ID NO : 13)
   Séquence 196 à 211 : SLEEFLKVTLRSTRQT (SEQ ID NO : 14).

Ainsi, de préférence, le polypeptide défini ci-dessus comprend au moins 5, 6, 7, 8, 9, 10, 11 ou 12 acides aminés, ou la totalité, ou est constitué de 5, 6, 7, 8, 9, 10, 11 ou 12 acides aminés, ou de la totalité, d'une séquence d'IL-6 sélectionné dans le groupe constitué des séquences :
- RYIIDGISALRKETCNKSNMC,
- NKSNMCESSKEALAENNLNLPK,
- AEKDGCFQSGFNEETC,
- FEVYLEYLQNRFESSEEQAR,
- NASLLTKLQAQNQWLQDM, et
- RSFKEFLQSSLRALRQM.

Par ailleurs, le polypeptide selon l'invention peut comprendre plusieurs répétitions, par exemple 2, 3, 4, 5, 10 ou 20 répétitions, respectivement de la séquence d'IL-6 ou d'IL-6R ou de la séquence variante définie ci-dessus.

### Séquence variante

Une séquence variante selon l'invention, qui a au moins 75% d'identité avec la séquence d'IL-6 ou d'IL-6R selon l'invention, aura de manière préférentielle au moins 80%, 85%, 90%, 95%, ou 98% d'identité avec la séquence d'IL-6 ou d'IL-6R selon l'invention. De manière très préférentielle, cette séquence variante aura au plus 50, 45, 40, 35, 30, 25, 20, 19, 18, 17, 16, 15, 14, 13, 12, 11 ou 10 acides aminés.

### Cyclisation

De préférence, le polypeptide selon l'invention est cyclisé.

Le polypeptide selon l'invention peut-être cyclisé, c'est à dire que l'ensemble du polypeptide forme un cycle ou qu'une portion de celui-ci forme un cycle, selon des méthodes de tout type connues de l'homme du métier.

En fonction des groupes fonctionnels présents dans le polypeptide, cette cyclisation peut s'effectuer de plusieurs manières différentes, comme par exemple : de son extrémité C-terminale à extrémité N-terminale, de son extrémité N-terminale à une chaîne latérale, d'une chaîne latérale à son extrémité C-terminale ou encore entre deux chaines latérales. Les groupements des chaines latérales impliqués dans la cyclisation sont notamment les groupes -NH₂, -COOH et -SH.

Parmi les diverses modalités de cyclisation de polypeptides, on peut citer la formation d'une liaison peptidique entre les deux extrémités, N- et C-terminales du polypeptide, la lactamisation, la lactonisation ou la formation d'un pont disulfure entre deux cystéine (C) du polypeptide. En particulier, lors de la formation d'un pont disulfure inter-cystéine, c'est-à-dire entre les radicaux -SH de deux cystéines, les cystéines peuvent être déjà présentes dans la séquence d'IL-6 ou d'IL-6R, ou bien être ajoutées au sein de ces séquences, pour former des séquences variantes, ou à leur(s) extrémité(s) N-terminale et/ou C-terminale.

### Modifications

Le polypeptide selon l'invention peut comprendre des modifications post-traductionnelles, telles que des glycosylations, des méthylations, des acylations, notamment par des acides gras ou par un groupe acétyle, des amidations, ou des phosphorylations. Par exemple, l'extrémité N-terminale du polypeptide selon l'invention peut être acétylée ou son extrémité C-terminale peut être modifiée par amidation.

### Séquences additionnelles

De préférence, le polypeptide selon l'invention comprend une ou plusieurs séquences additionnelles, en plus de la séquence de l'IL-6 ou de l'IL-6R selon l'invention.

Ces séquences additionnelles selon l'invention peuvent notamment apporter des caractéristiques physico-chimiques permettant une présentation structurale améliorée ou une solubilité améliorée du polypeptide selon l'invention par rapport à un polypeptide similaire mais qui ne comprendrait pas ces séquences additionnelles. Les séquences additionnelles selon l'invention peuvent notamment comprendre une ou plusieurs séquences de lien, ou de liaison, peptidique (« peptide linker »), utiles pour une liaison notamment à une molécule porteuse. De telles séquences de lien peptidique comprennent typiquement de 1 à 10, notamment de 1 à 6, et en particulier 2 à 5 acides aminés.

Une séquence de lien particulièrement préféré selon l'invention est la séquence EGEX (SEQ ID NO : 15), où X est un acide aminé permettant la liaison à la protéine porteuse, notamment choisi dans le groupe constitué de cystéine (C), tyrosine (Y), et lysine (K). Avantageusement, la séquence EGEX améliore la solubilité tout en maintenant de bonnes réponses immunitaires comme les Exemples 4, 5, et 6 ci-après le montrent.

Par ailleurs, la ou les séquences additionnelles peuvent aussi comprendre des épitopes appartenant à d'autres protéines que l'IL-6 ou l'IL-6R, permettant d'éliciter ou de générer une réponse immunitaire dirigée contre ces autres protéines.

En outre, les séquences additionnelles selon l'invention peuvent également comprendre des séquences d'épitope(s) T exogène(s), préférablement universel(s). Avantageusement, ces séquences d'épitopes T additionnelles permettent de renforcer l'immunogénicité du polypeptide selon l'invention.

### Préparation

Le polypeptide selon l'invention peut être préparé par toute méthode connue dans l'état de la technique et notamment par synthèse chimique. Il est également possible de le préparer par la voie recombinante dans des cellules eucaryotes ou procaryotes.

### Protéine porteuse

La protéine porteuse selon l'invention est une toxine diphtérique mutante non-toxique, c'est-à-dire une anatoxine diphtérique obtenue par mutagenèse. Avantageusement, cette toxine diphtérique mutante est pharmaceutiquement acceptable.

La protéine porteuse selon l'invention est de préférence choisie dans le groupe constitué de CRM197, de CRM176, de CRM228, de CRM45, de CRM9, de CRM102, de CRM103, et de CRM107.

De manière particulièrement préférée, la protéine porteuse selon l'invention est CRM197.

CRM197 est une forme génétiquement détoxifiée de la toxine diphtérique. Elle possède une mutation unique en position 52, substituant une glycine (G) par un acide glutamique (E), ce qui provoque la perte de l'activité ADP-ribosyltransférase. Elle conserve en revanche tous les radicaux amines des lysines (K) disponibles pour la conjugaison. CRM197 possède 535 acides aminés (58,4 kDa) et consiste en deux sous-unités reliées par des ponts disulfures. Elle est décrite sous le numéro d'accession GenBank 1007216A (SEQ ID NO : 16). Elle peut être produite de manière recombinante chez *Corynebacterium diphtheria,* et également par d'autres bactéries telles que *Pseudomonas fluorescens* ou *Escherichia coli.*

### Agent de couplage

De préférence l'agent de couplage selon l'invention est non peptidique.

L'agent de couplage peut être hétérobifonctionnel, tel que l'ester de N-γ-maleimidobutyryl-oxysuccinimide (GMBS) et le derivé sulfo-GMBS, l'ester du m-maléimidobenzoyl-n-hydroxysuccinimide (MBS) et le derivé sulfo-MBS, le succinimidyl 4-(N-maleimidomethyl)cyclohexane-1-carboxylate (SMCC), le sulfo-succinimidyl 4-(N-maleimidomethyl)cyclohexane-1-carboxylate (sulfo-SMCC), un carbodiimide, le bisdiazonium-benzidine (BDB) ou le glutaraldéhyde. L'utilisation des agents de couplage est notamment décrite dans le chapitre *"*Production of Antisera Using Peptide Conjugates" de l'ouvrage de référence "The Protein Protocols Handbook" (2002) qui est incorporé ici par référence.

Lorsque le GMBS, le MBS, le SMCC ou le sulfo-SMCC sont utilisés, ils sont de préférence fixés sur une cystéine (C), qui si elle n'est pas présente dans la séquence du peptide, peut être ajoutée, notamment à son extrémité N-terminale ou C-terminale.

### Conjugué

De préférence, le polypeptide du conjugué immunogène selon l'invention comprend, ou est constitué de, la séquence ESKEALAENNLNLPK (SEQ ID NO : 17), plus particulièrement la séquence EESKEALAENNLNLPK (SEQ ID NO: 18), et encore plus particulièrement la séquence EESKEALAENNLNLPKC (SEQ ID NO: 19), la séquence AEESKEALAENNLNLPKC (SEQ ID NO : 20) ou la séquence CEESKEALAENNLNLPKC (SEQ ID NO : 21), à laquelle est éventuellement ajoutée, du côté C-terminal, la séquence additionnelle de lien peptidique EGEX définie ci-dessus. De préférence également ce polypeptide est lié à la protéine porteuse CRM197 définie ci-dessus par un agent de couplage défini ci-dessus.

Ainsi, de préférence, le conjugué immunogène tel que défini ci-dessus est de formule (I) suivante :

[(AEESKEALAENNLNLPKC)-GMB]-CRM197 (I)

dans laquelle :
- le polypeptide est constitué de la séquence AEESKEALAENNLNLPKC,
- la protéine porteuse est CRM197,
- le polypeptide est lié de manière covalente à la protéine porteuse via l'agent de couplage N-γ-maléimidobutyryl- (GMB),
- les parenthèses indiquent que le polypeptide est cyclisé par formation d'une liaison peptidique entre la cystéine (C) C-terminale et l'alanine (A) N-terminale,
- les crochets indiquent qu'au moins un polypeptide est lié à la protéine porteuse.

De préférence également, le conjugué immunogène tel que défini ci-dessus est de formule (II) suivante :

[Acétyl-(CEESKEALAENNLNLPKC)-(X)-C-GMB]-CRM197, (II)

dans laquelle :
- le polypeptide est constitué de la séquence CEESKEALAENNLNLPKC-(X)ᵢ-C
- la protéine porteuse est CRM197,
- le polypeptide est lié de manière covalente à la protéine porteuse via l'agent de couplage N-γ-maléimidobutyryl- (GMB),
- les parenthèses indiquent que la partie de séquence CEESKEALAENNLNLPKC du polypeptide est cyclisée par formation d'un pont disulfure entre la cystéine (C) C-terminale et la cystéine (C) N-terminale,
- Acétyl- indique que la fonction -NH₂ libre de la cystéine (C) N-terminale est protégée par un groupement acétyl par formation d'une liaison amide,
- (X) représente une séquence peptidique quelconque de 1 à 5 acides aminés, en particulier EGE,
- i vaut 0 ou 1
- les crochets indiquent qu'au moins un polypeptide est lié à la protéine porteuse.

### Composition pharmaceutique, notamment vaccinale

Parmi les adjuvants qui peuvent être administrés conjointement avec le conjugué immunogène défini ci-dessus ou qui peuvent être présent dans la composition pharmaceutique, notamment vaccinale, définie ci-dessus on peut citer l'Alun (hydroxyde d'alumine), le MONTANIDE™ ISA 51 VG, le MONTANIDE™ ISA 720 VG, toute émulsion eau dans huile ou toute émulsion huile dans eau, ainsi que de manière générale tous les adjuvants connus dans la littérature scientifique.

Il est également possible d'administrer conjointement avec le conjugué immunogène selon l'invention ou d'ajouter à la composition pharmaceutique, notamment vaccinale, selon l'invention, des immuno-modulateurs, comme le MP40 par exemple.

### Applications thérapeutiques

Le conjugué immunogène selon l'invention ou la composition pharmaceutique, notamment vaccinale, le comprenant, est à visée d'immunisation active et peut être administrées pour soigner toutes les maladies liées à une surproduction ou une surexpression de la cytokine inflammatoire IL-6 en induisant la production d'anticorps qui se lieront à l'IL-6 surproduite ou surexprimé pour l'empêcher d'agir.

De préférence, les maladies liées à la surproduction ou à la surexpression de l'IL-6 selon l'invention sont sélectionnées dans le groupe constitué de :
- Les maladies inflammatoires chroniques de l'intestin comme la maladie de Crohn et la rectocolite hémorragique ou la colite ulcéreuse ;
- Les maladies arthritiques comme la polyarthrite rhumatoïde, l'arthrite juvénile, le psoriasis arthritique, l'arthrose, la polyarthrite rhumatoïde réfractaire, l'arthrite chronique non rhumatoïde, la spondylarthrite ankylosante ;
- Les maladies inflammatoires osseuses chroniques ou liées à IL-6, en particulier l'ostéoporose ou toute maladie de résorption de l'os ;
- Les maladies inflammatoires chroniques ou liées à IL-6 associées à une infection comme le choc septique, le choc endotoxinique, la septicémie, l'hépatite C, le paludisme, le SIDA ou d'autres infections liées au VIH ;
- Les maladies inflammatoires chroniques ou liées à l'IL-6 du système cardio-vasculaire, comme l'atherosclérose, les lésions liées à la reperfusion suite à une ischémie, les maladies coronariennes, les vascularites, comme la maladie de Behcet ou la granulomatose de Wegener ;
- Les maladies auto-immunes comme la sclérodermie, la sclérodermie systémique, le lupus eryhtémateux, en particulier disséminé, la sclérose en plaques, ou le psoriasis ;
- Les maladies liées aux greffes, notamment les réactions de type greffon contre l'hôte, les rejets de greffe, et les traumatismes ;
- Les allergies, en particulier l'asthme allergiques, et les désordres cutanés liés à des réactions d'hypersensibilité retardée ;
- Les immunodéficiences, comme l'immunodéficience idiopathique (CVID en anglais) ;
- Les maladies inflammatoires chroniques ou liées à IL-6 de l'appareil respiratoire, en particulier le syndrome de détresse respiratoire ou la fibrose pulmonaire ;
- Les cancers qui ont une composante inflammatoire chronique ou liée à IL6, comme le plamocytome, le cancer colo-rectal, le cancer de l'ovaire, les syndromes lymphoprolifératifs, le myélome multiple, en particulier le myélome multiple réfractaire, ou les syndromes myéloprolifératifs ;
- Le diabète, en particulier le diabète juvénile ;
- Les amyloses, en particulier la maladie d'Alzheimer ;
- Les uvéites, en particulier leur forme récurrente ;
- La cachexie ; et
- L'endométriose.

### Administration

De préférence la quantité de conjugué immunogène selon l'invention administré par administration, c'est-à-dire la dose unitaire de conjugué immunogène administrée, est comprise entre 1 nanogramme et 1 g, plus préférablement entre 1 microgramme et 1 milligramme.
L'administration du conjugué immunogène est possible par voie intraveineuse, intradermique, sous-cutanée, intra-musculaire, mucosale, notamment intra-nasale, ou intra-péritonéale.
Le régime d'administration ou posologique peut aller d'une administration tous les 15 jours à une fois par an pour l'amorçage (priming), jusqu'à ce qu'une bonne réponse anticorps anti-IL-6 ou anti-IL-6R apparaisse, puis avec des rappels espacés par exemple tous les 2 mois à une fois par an pour que les anticorps gardent une activité bénéfique satisfaisante.
De préférence, l'individu selon l'invention est un animal, notamment un mammifère, en particulier un humain. De préférence, l'individu selon l'invention surproduit ou surexprime l'IL-6 ou est à risque de surproduire ou de surexprimer l'IL-6

L'invention sera davantage explicitée à l'aide des exemples non limitatifs qui suivent.

### EXEMPLES

### Exemple 1

Le peptide (CESSKEALAENNLNLPKC)Y (SEQ ID NO : 22), synthétisé chimiquement, est conjugué à la protéine porteuse CRM197 (Pfenex, USA) d'une part et à la protéine porteuse KLH (*Keyhole limpet hemocyanin*, SIGMA) d'autre part.
Le peptide correspond à la zone 78-93 de l'IL-6 humaine (partie soulignée ci-dessus) avec ajout d'une cystéine et d'une tyrosine en C-terminal. La cystéine permet une cyclisation (partie entre parenthèses) avec la cystéine présente en N-terminal du peptide via un pont disulfure. La conjugaison aux protéines porteuses est réalisée via la tyrosine avec de la bis-diazo-benzidine (BDB, PolyPeptides, Strasbourg France) selon des méthodes standards.
La production de ces conjugués conduit généralement à un rapport massique standard peptide/protéine porteuse de l'ordre de 1:2 pour le KLH et pour la CRM197 comme déterminé par la méthode de dosage des acides aminés *Amino-Acid Analysis* (AAA). En d'autres termes, il est déterminé que pour 300 microgrammes de masse totale de peptide conjugué à la protéine porteuse, il y a 200 microgrammes en KLH et 100 microgrammes en peptide, et il y a 200 microgrammes en CRM197 et 100 microgrammes en peptide pour le conjugué à CRM197.
Ces conjugués sont testés dans une expérience d'immunisation chez la souris Swiss (Charles River Laboratoires, Ecully, France) en utilisant pour adjuvant l'ISA51 (SEPPIC, France), en administrant un mélange contenant 50 microlitres d'ISA51 pour 50 microlitres de conjugué, par voie intramusculaire, avec une dose injectée de 300 microgrammes de conjugué pour chaque immunisation. Deux groupes de 8 souris sont ainsi immunisés à J0, J14, J28, J42 contre le peptide conjugué au KLH et contre le peptide conjugué à la CRM197. Les souris sont sacrifiées à J54, leur sang prélevé, et les sera ainsi préparés sont évalués pour leurs taux d'anticorps anti-IL-6 humaine mesurés par ELISA (Desallais et al. (2016) Sci. Rep. 6:19549). Au jour du sacrifice, les souris se portent bien (poil bien luisant, poids conservé, selles normales), ce qui montre que l'immunisation contre les conjugués immunogène n'a pas d'effet toxique à la dose utilisée.

Les titres en anticorps anti-IL6 obtenus pour les 2 groupes, KLH et CRM197, sont résumés dans le tableau ci-après :

| | CRM197 | KLH |
|---|---|---|
| Moyenne titres | 20000 | 4788 |
| Médiane titres | 10980 | 1608 |

On voit que pour des doses en conjugués identiques, les titres d'anticorps sont nettement supérieurs avec le conjugué CRM197 par rapport au conjugué KLH.

### Exemple 2

Les mêmes composés que pour l'Exemple 1 sont testés lors d'immunisations en sous-cutané de singes macaques réalisées à J1, J15, J30, J45, avec pour adjuvant l'ISA51, en un mélange 250 microlitres d'ISA51/250 microlitres de conjugué. 300 microgrammes de conjugué au KLH et 300 microgrammes de conjugué à la CRM197 sont administrés lors de chaque immunisation respectivement à deux groupes de 4 animaux.
Des prélèvements de sang et préparation de sérum sont faits à J57, et les titres d'anticorps dirigés contre l'IL-6 humaine mesurés par ELISA. Au jour du sacrifice, les souris se portent bien (poil bien luisant, poids conservé, selles normales), ce qui montre que l'immunisation contre les conjugués immunogène n'a pas d'effet toxique à la dose utilisée.

Les titres en anticorps anti-IL6 obtenus pour les 2 groupes, KLH et CRM197, sont résumés dans le tableau ci-après :

| | CRM197 | KLH |
|---|---|---|
| Moyenne titres | 20510 | 7498 |
| Médiane titres | 17514 | 12116 |

On voit à nouveau que les titres obtenus après immunisation contre le conjugué CRM197 sont nettement plus élevés en moyenne et en médiane que ceux obtenus pour le KLH.

### Exemple 3

Le peptide cyclo(LTKLQAQNQWLQDMC) (SEQ ID NO : 23), synthétisé chimiquement, est conjugué à la protéine porteuse CRM197 ou KLH via le groupe -SH de la cystéine.

La conjugaison est réalisée avec l'agent de couplage bi-fonctionnel N-γ-maleimidobutyryl succinimide (GMBS, Thermo-Fisher 22309) selon des méthodes standards (« *User guide GMBS and SulfoGMBS* » Thermo-Fisher). Le peptide comprend la séquence 176-189 de l'IL-6 humaine (partie soulignée ci-dessus), à laquelle est ajoutée une cystéine en C-terminal, et est cyclisé par lactamisation (partie entre parenthèses ci-dessus).
La production de ces conjugués conduit à un ratio massique standard peptide/protéine porteuse estimé de 1:2 pour le KLH et 1:2 pour la CRM197. En d'autres termes, il est déterminé que pour 300 microgrammes de masse totale de peptide conjugué, il y a 200 microgrammes en KLH et 100 microgrammes en peptide pour le conjugué au KLH, et de même pour le conjugué à la CRM197 (100 microgrammes en peptide pour 200 microgrammes en CRM197).
Ces conjugués sont testés dans une expérience d'immunisation chez la souris en utilisant pour adjuvant, l'ISA51 (50 microlitres d'ISA51 pour 50 microlitres de conjugué), par voie intramusculaire avec une dose de 450 microgrammes de conjugué pour chaque immunisation. 2 groupes de 8 souris sont immunisés à J0, J14, J28, J42 contre le peptide conjugué au KLH et contre le peptide conjugué à la CRM197. Les souris sont sacrifiées à J54, leursang prélevé, et les sera ainsi préparés sont évalués pour leurs taux d'anticorps anti-IL-6 humain mesurés par ELISA. Au jour du sacrifice, les souris se portent bien (poil bien luisant, poids conservé, selles normales), ce qui montre que l'immunisation contre les conjugués immunogène n'a pas d'effet toxique à la dose utilisée.

Les titres obtenus pour les 2 groupes, KLH et CRM197, sont résumés dans le tableau ci-après :

| | CRM197 | KLH |
|---|---|---|
| Moyenne titres | 12350 | 8480 |
| Médiane titres | 7608 | 4772 |

On voit que pour des doses en conjugués identiques, les titres d'anticorps sont encore une fois nettement supérieurs avec le conjugué CRM197 par rapport au conjugué KLH.

### Exemple 4

Le peptide (CIDKQIRYIIDGISALRKETC)EGEC (SEQ ID NO : 24), synthétisé chimiquement, est conjugué à la protéine porteuse CRM197 d'une part ou KLH d'autre part. Le peptide comprend la séquence 53 à 71 de l'IL-6 humaine (partie soulignée ci-dessus) encadrée par deux cystéines qui permettent la cyclisation du peptide par formation d'un pont disulfure (partie entre parenthèse ci-dessus). La conjugaison est réalisée via la cystéine C-terminale à l'aide de l'agent de couplage bi-fonctionnel N-γ-maleimidobutyryl succinimide (GMBS). Un peptide de liaison (linker) de séquence EGEC est présent.
La production de ces conjugués conduit à un rapport massique standard peptide/protéine porteuse estimé de 1:2 pour le KLH et 1:2 pour la CRM197. En d'autres termes, il est déterminé que pour 300 microgrammes de masse totale de peptide conjugué, il y a 200 microgrammes en KLH et 100 microgrammes en peptide pour le conjugué au KLH, et de même pour le conjugué à la CRM197 (100 microgrammes en peptide pour 200 microgrammes en CRM197).
Ces conjugués sont testés lors d'immunisations de deux groupes de 6 lapins New Zealand White (NZW) (Charles River) réalisées à J1, J15, J45, avec pour adjuvant l'ISA51, en sous-cutané. Dans le cas présent les lapins sont immunisés contre 450 microgrammes de conjugué (peptide-KLH ou bien peptide-CRM197) lors de chaque immunisation. Le volume injecté en ISA51 est de 400 microlitres (200 microlitres d'ISA51 avec 200 microlitres de conjugué).
Des prélèvements de sang et la préparation de sérum à partir de ces prélèvements sont faits à J60. Les titres d'anticorps dirigés contre l'IL-6 humaine sont mesurés par ELISA. Au jour du sacrifice, les souris se portent bien (poil bien luisant, poids conservé, selles normales), ce qui montre que l'immunisation contre les conjugués immunogène n'a pas d'effet toxique à la dose utilisée.

Les titres obtenus pour les 2 groupes, KLH et CRM197, sont résumés dans le tableau ci-après :

| | CRM197 | KLH |
|---|---|---|
| Moyenne titres | 11192 | 8756 |
| Médiane titres | 6328 | 5640 |

On voit à nouveau que les titres obtenus après immunisation contre le conjugué CRM197 sont nettement plus élevés en moyenne et en médiane que ceux obtenus pour le KLH.

### Exemple 5

Les peptides (A) (CLQAQNQWLQDMC)Y (SEQ ID NO : 25) et (B) (CLQAQNQWLQDMC)EGEY (SEQ ID NO: 26), synthétisés chimiquement, sont conjugués à la protéine porteuse CRM197. Le peptide comprend la séquence s'étendant des résidus 179 à 189 de l'IL-6 humaine (partie soulignée ci-dessus), avec des cystéines ajoutées aux extrémités N- et C-terminale pour la cyclisation. Le peptide est cyclisé par formation d'un pont disulfure entre les cystéines (partie soulignée), et le couplage à la CRM197 de ces peptides est réalisé par la tyrosine terminale à l'aide L'agent de couplage bi-fonctionnel utilisé est le BDB. Le peptide (B) est identique au peptide (A) à l'exception d'une séquence de liaison (linker) constituée du tripeptide de séquence EGE.
La production de ces conjugués conduit à un rapport massique standard peptide/protéine porteuse estimé de 1:2. En d'autres termes, il est déterminé que pour 300 microgrammes de poids total de conjugué, il y a 200 microgrammes de CRM197 et 100 microgrammes de peptide.
Ces composés sont testés lors d'immunisations de deux groupes de 6 souris réalisées à J1, J15, J45, avec pour adjuvant l'ISA51, en sous-cutané. Dans le cas présent les souris sont immunisées contre 300 microgrammes de conjugué lors de chaque immunisation. Le volume injecté en ISA51 est de 200 microlitres (100 microlitres d'ISA51 avec 100 microlitres de conjugué).
Des prélèvements de sang et la préparation de sérum à partir de ces prélèvements sont faits à J54. Les titres d'anticorps dirigés contre l'IL-6 humaine sont mesurés par ELISA. Au jour du sacrifice, les souris se portent bien (poil bien luisant, poids conservé, selles normales), ce qui montre que l'immunisation contre les conjugués immunogène n'a pas d'effet toxique à la dose utilisée.

Les titres obtenus pour les 2 groupes sont résumés dans le tableau ci-après :

| | Avec peptide de liaison EGEY | Sans peptide de liaison |
|---|---|---|
| Moyenne des titres | 3675 | 2718 |
| Médiane des titres | 1752 | 1146 |

On voit que les titres obtenus après immunisation contre le peptide possédant le peptide de liaison EGEY sont nettement plus élevés en moyenne et en médiane que ceux obtenus pour le peptide sans peptide de liaison.

### Exemple 6

Les peptides (A) (CFQSGFNEETC)Y (SEQ ID NO : 27) et (B) (CFQSGFNEETC)EGEY (SEQ ID NO : 28), synthétisés chimiquement, sont conjugués à la protéine porteuse CRM197. Les peptides comprennent la séquence 101 à 111 de l'IL-6 humaine (partie soulignée ci-dessus, les cystéines (C) sont natives). Le peptide est cyclisé par formation d'un pont disulfure entre les deux cystéines. Le couplage à la CRM197 de ces peptides est réalisé par la tyrosine terminale à l'aide de l'agent de couplage bi-fonctionnel BDB.
La production de ces conjugués conduit à un rapport massique standard peptide/protéine porteuse estimé de 1:2. En d'autres termes, il est déterminé que pour 300 microgrammes de poids total de conjugué, il y a 200 microgrammes de CRM197 et 100 microgrammes de peptide.
Ces composés sont testés lors d'immunisations de deux groupes de 6 souris réalisées à J1, J15, J45, avec pour adjuvant l'ISA51, en sous-cutané. Dans le cas présent les souris sont immunisées contre 300 microgrammes de conjugué lors de chaque immunisation. Le volume injecté en ISA51 est de 200 microlitres (100 microlitres ISA51 avec 100 microlitres de conjugué).
Des prélèvements de sang et la préparation de sérum à partir de ces prélèvements sont faits à J54. Les titres d'anticorps dirigés contre l'IL-6 humaine sont mesurés par ELISA. Au jour du sacrifice, les souris se portent bien (poil bien luisant, poids conservé, selles normales), ce qui montre que l'immunisation contre les conjugués immunogène n'a pas d'effet toxique à la dose utilisée.

Les titres obtenus pour les 2 groupes sont résumés dans le tableau ci-après :

| | Avec peptide de liaison EGEY | Sans peptide de liaison |
|---|---|---|
| Moyenne des titres | 2648 | 980 |
| Médiane des titres | 1083 | 368 |

On voit à nouveau que les titres obtenus après immunisation contre le peptide possédant le peptide de liaison EGEY sont nettement plus élevé en moyenne et en médiane que ceux obtenus pour le peptide sans peptide de liaison.

**Récapitulatif des identifiants de séquence :**

| **SEQ ID NO :** | **Description** |
|---|---|
| 1 | IL-6 humaine |
| 2 | Sous-unité alpha du récepteur de l'IL-6 humaine |
| 3 | RYIIDGISALRKETCNKSNMC |
| 4 | NKSNMCESSKEALAENNLNLPK |
| 5 | AEKDGCFQSGFNEETC |
| 6 | FEVYLEYLQNRFESSEEQAR |
| 7 | NASLLTKLQAQNQWLQDM |
| 8 | RSFKEFLQSSLRALRQM |
| 9 | VLWEIVEMRKELCNGNSDCMN |
| 10 | NSDCMNNDDALAENNLKLPEIG |
| 11 | NDGCYQTGYNQEICLL |
| 12 | SYLEYMKNNLKDNKKDKARV |
| 13 | ALLTDKLESQKEWLRTKT |
| 14 | SLEEFLKVTLRSTRQT |
| 15 | EGEX |
| 16 | CRM197 |
| 17 | ESKEALAENNLNLPK |
| 18 | EESKEALAENNLNLPK |
| 19 | EESKEALAENNLNLPKC |
| 20 | AEESKEALAENNLNLPKC |
| 21 | CEESKEALAENNLNLPKC |
| 22 | CESSKEALAENNLNLPKCY |
| 23 | LTKLQAQNQWLQDMC |
| 24 | CIDKQIRYIIDGISALRKETCEGEC |
| 25 | CLQAQNQWLQDMCY |
| 26 | CLQAQNQWLQDMCEGEY |
| 27 | CFQSGFNEETC |
| 28 | CFQSGFNEETCEGEY |

## Revendications

1. Conjugué immunogène comprenant :
- une protéine porteuse et
- au moins un polypeptide ayant au plus 100 acides aminés comprenant une séquence de 5 à 50 acides aminés de l'interleukine 6 (IL-6) ou du récepteur de l'interleukine 6 (IL-6R), ou une séquence variante présentant au moins 75% d'identité avec la séquence de 5 à 50 acides aminés de l'IL-6 ou de l'IL-6R,
dans lequel le polypeptide est lié de manière covalente à la protéine porteuse et la protéine porteuse est une toxine diphtérique mutante non-toxique.

2. Conjugué immunogène selon la revendication 1, dans lequel la protéine porteuse est CRM197.

3. Conjugué immunogène selon la revendication 1 ou 2, dans lequel le polypeptide comprend une séquence de 7 à 35 acides aminés de l'IL-6 ou de l'IL-6R ou une séquence variante présentant au moins 90% d'identité avec la séquence de 7 à 35 acides aminés de l'IL-6 ou de l'IL-6R.

4. Conjugué immunogène selon l'une des revendications 1 à 3, dans lequel le polypeptide est cyclisé.

5. Conjugué immunogène selon l'une des revendications 1 à 4, dans lequel le polypeptide comprend aux moins 5 acides aminés d'une séquence sélectionnée dans le groupe constitué des séquences :
• RYIIDGISALRKETCNKSNMC,
• NKSNMCESSKEALAENNLNLPK,
• AEKDGCFQSGFNEETC,
• FEVYLEYLQNRFESSEEQAR,
• NASLLTKLQAQNQWLQDM, et
• RSFKEFLQSSLRALRQM.

6. Conjugué immunogène selon l'une des revendications 1 à 5, dans lequel le polypeptide est lié à la protéine porteuse via un agent de couplage non peptidique.

7. Conjugué immunogène selon l'une des revendications 1 à 6, de formule (I) suivante :
[(AEESKEALAENNLNLPKC)-GMB]-CRM197 (I)
dans laquelle :
• le polypeptide est constitué de la séquence AEESKEALAENNLNLPKC,
• la protéine porteuse est CRM197,
• le polypeptide est lié de manière covalente à la protéine porteuse via l'agent de couplage N-γ-maléimidobutyryl- (GMB),
• les parenthèses indiquent que le polypeptide est cyclisé par formation d'une liaison peptidique entre la cystéine (C) C-terminale et l'alanine (A) N-terminale,
• les crochets indiquent qu'au moins un polypeptide est lié à la protéine porteuse.

8. Conjugué immunogène selon l'une des revendications 1 à 6, de formule (II) suivante :
[Acétyl-(CEESKEALAENNLNLPKC)-(X)ᵢ-C-GMB]-CRM197, (II)
dans laquelle :
• le polypeptide est constitué de la séquence CEESKEALAENNLNLPKC-(X)ᵢ-C
• la protéine porteuse est CRM197,
• le polypeptide est lié de manière covalente à la protéine porteuse via l'agent de couplage N-γ-maléimidobutyryl- (GMB),
• les parenthèses indiquent que la partie de séquence CEESKEALAENNLNLPKC du polypeptide est cyclisée par formation d'un pont disulfure entre la cystéine (C) C-terminale et la cystéine (C) N-terminale,
• Acétyl- indique que la fonction -NH₂ libre de la cystéine (C) N-terminale est protégée par un groupement acétyl par formation d'une liaison amide,
• (X) représente une séquence peptidique quelconque de 1 à 5 acides aminés
• i vaut 0 ou 1
• les crochets indiquent qu'au moins un polypeptide est lié à la protéine porteuse.

9. Conjugué immunogène selon l'une des revendications 1 à 8, pour une utilisation dans une méthode de traitement thérapeutique ou dans une méthode de prévention ou de traitement d'une maladie liée à une surproduction de l'IL-6, chez un individu.

10. Conjugué immunogène selon l'une des revendications 1 à 8, pour une utilisation dans une méthode de vaccination contre l'IL-6 ou l'IL-6R, ou dans une méthode d'induction d'une réponse immunitaire contre l'IL-6 ou l'IL-6R, chez un individu.

11. Composition pharmaceutique, notamment vaccinale, comprenant à titre de substance active au moins un conjugué immunogène tel que défini dans l'une des revendications 1 à 8, éventuellement en association avec au moins un véhicule et/ou excipient pharmaceutiquement acceptable.

12. Composition pharmaceutique, notamment vaccinale, selon la revendication 11, comprenant en outre au moins un adjuvant.

13. Composition pharmaceutique, notamment vaccinale, selon la revendication 11 ou 12, pour une utilisation dans une méthode de traitement thérapeutique ou une méthode de prévention ou de traitement d'une maladie liée à une surproduction de l'IL-6, chez individu.

14. Composition pharmaceutique, notamment vaccinale, selon la revendication 11 ou 12, pour une utilisation dans une méthode de vaccination contre l'IL-6 ou l'IL-6R, ou dans une méthode d'induction d'une réponse immunitaire contre l'IL-6 ou l'IL-6R, chez un individu.

15. Procédé de préparation d'un conjugué immunogène tel que défini dans l'une des revendication 1 à 8, comprenant une étape de liaison covalente d'au moins un polypeptide ayant au plus 100 acides aminés comprenant une séquence de 5 à 50 acides aminés de l'interleukine 6 (IL-6) ou du récepteur de l'IL-6 (IL-6R), ou une séquence variante présentant au moins 75% d'identité avec la séquence de 5 à 50 acides aminés de l'IL-6 ou de l'IL-6R avec une protéine porteuse qui est une toxine diphtérique mutante non-toxique.
